# FASCICULE DE BREVET EUROPEEN

(11) **EP 1 718 211 B1**
(45) Date de publication et mention de la délivrance du brevet: **14.05.2008**
(21) Numéro de dépôt: 05732462.6
(22) Date de dépôt: 25.02.2005
(51) Int. Cl.: A61B 8/10

(54) **SONDE ECHOGRAPHIQUE A BALAYAGE SECTORIEL UTILISANT UN TRANSDUCTEUR APTE A VENIR AU CONTACT DE LA STRUCTURE A EXAMINER**
SEKTOR-SCANNING-ULTRASCHALLSONDE UNTER VERWENDUNG EINES WANDLERS, DER MIT DER ZU UNTERSUCHENDEN STRUKTUR IN BERÜHRUNG KOMMEN KANN
SECTOR SCANNING ULTRASOUND PROBE THAT USES A TRANSDUCER WHICH CAN COME INTO CONTACT WITH THE STRUCTURE TO BE EXAMINED

(30) Priorité: 27.02.2004 FR 0402209
(43) Date de publication de la demande: 08.11.2006
(73) Titulaire: Quantel Medical, 63100 Clermond Ferrand (FR)
(72) Inventeur: ABASCAL, Jean, F-94110 Arcueil (FR)
(74) Mandataire: de Saint-Palais, Arnaud Marie
(86) Numéro de dépôt international: PCT/FR2005/000465
(87) Numéro de publication internationale: WO 2005/089652

(56) Documents cités:
- EP-A- 0 403 349
- WO-A-94/27501
- US-A- 3 753 219
- US-A- 5 357 963
- US-A- 5 487 388
- US-A- 5 630 416
- US-B1- 6 213 948
- US-B1- 6 287 261

## Description

La présente invention concerne une sonde échographique à balayage sectoriel utilisant un transducteur apte à venir au contact de la structure à examiner.

Elle s'applique notamment; mais non exclusivement, à l'échographie de structures oculaires.

D'une manière générale, on sait qu'en ophtalmologie, l'échographie 2-D à 10 MHz est utilisée en pratique courante pour l'exploration de l'anatomie et des pathologies des structures oculaires, et, plus particulièrement, celles du pôle postérieur (rétine, nerf optique, vitré).

La technique des sondes utilise un balayage sectoriel, ce qui permet d'obtenir des sondes de petites tailles comparativement à des sondes à barrettes.

Pour effectuer ce type de balayage, on utilise des sondes échographiques comportant un boîtier tubulaire ouvert dans sa partie antérieure et dont le volume intérieur est divisé en deux compartiments par une cloison étanche, à savoir :
- un compartiment postérieur qui s'étend du côté du fond du boîtier : ce compartiment comprend une motorisation et des circuits d'alimentation de commande et de traitement de l'appareil,
- un compartiment antérieur, adjacent à l'ouverture du boîtier, qui renferme un transducteur mobile ainsi que tout ou partie de son mécanisme d'actionnement.

Habituellement, l'ouverture du compartiment antérieur est refermée par une membrane étanche souple ou dure, de manière à obtenir un volume intérieur étanche renfermant un liquide de couplage qui doit avoir un haut pouvoir de transmission des ondes ultrasonores.

Une telle disposition est divulguée dans le brevet US 5 357 263 qui fait intervenir un transducteur rotatif logé dans le compartiment antérieur et entraîné depuis l'extérieur de ce compartiment grâce à une liaison à couplage magnétique.

Le brevet US 5 487 388 décrit un dispositif de balayage ultrasonique comprenant une sonde logeant dans son extrémité d'un transducteur revêtue d'une couche réalisée en un matériau assurant une bonne transmission des ondes ultrasonores, cette couche présentant une surface extérieure de révolution, la sonde étant montée rotative autour d'un axe de rotation.

La membrane, qui est destinée à venir en contact avec l'oeil du patient, doit être réalisée en une matière biocompatible qui n'atténue pas les ultrasons de haute fréquence. Elle confine le liquide de couplage tout en permettant de protéger l'oeil de tout contact accidentel avec des pièces mécaniques, à savoir notamment, le transducteur et/ou son mécanisme d'actionnement. Or, ce sont des résultats difficiles à obtenir, ce qui explique l'intérêt de l'invention de ramener ces problèmes à la réalisation du transducteur lui-même, c'est-à-dire à la source des ultrasons, de manière à pouvoir agir plus facilement sur les caractéristiques globales du transducteur.

Il s'avère que les appareils échographiques de ce type présentent néanmoins un certain nombre d'inconvénients. En effet :

Avant de parvenir à l'oeil, l'onde ultrasonore focalisée par la courbure concave de l'élément piézoélectrique du transducteur, doit traverser plusieurs couches de matière présentant des propriétés différentes et ce, avec des vitesses de propagation différentes, le trajet de ces ondes à l'intérieur de ces couches variant en fonction de la position (variable) du transducteur.

Ceci provoque des variations de la distance focale du transducteur et des imprécisions dans l'image échographique obtenue.

Par ailleurs, on constate que, dans le cas de transducteur hautes fréquences (à partir de 15 MHz), les liquides et membranes deviennent de plus en plus absorbants. De ce fait, les fréquences pénétrant effectivement dans les tissus sont bien inférieures aux fréquences émises par le transducteur.

Ainsi, à titre d'exemple, pour un transducteur émettant à une fréquence de 20 MHz, la fréquence centrale transmise sera de 18 MHz avec une atténuation de 10 dB.

Dans le cadre des sondes ouvertes, tous ces inconvénients sont supprimés car le milieu de couplage consiste en un bain d'eau possédant de bonnes propriétés acoustiques. Cependant, le transducteur effectue son mouvement à une très faible distance de la structure à examiner, et même s'il est de forme circulaire, ses bords sont agressifs et, en conséquence, il n'est pas possible de supprimer tout risque de traumatisme par contact accidentel du transducteur avec l'oeil (éraflement de la cornée par l'arête circulaire du transducteur).

L'invention a donc plus particulièrement pour but de supprimer ces inconvénients.

Elle propose, à cet effet, une sonde échographique à balayage sectoriel comprenant un corps tubulaire logeant au moins partiellement dans son extrémité antérieure, un transducteur conçu de manière à émettre une onde ultrasonore incidente focalisée en direction des structures à examiner, et à recevoir des ondes ultrasonores engendrées par ces structures sous l'effet de cette onde incidente, ce transducteur étant associé à des moyens d'actionnement de manière à pouvoir effectuer des déplacements, au moins partiellement en rotation en vue d'obtenir un balayage sectoriel des structures à examiner.

Selon l'invention, le transducteur comprend un élément piézoélectrique présentant un pouvoir de focalisation des faisceaux émis et dont la partie antérieure est revêtue d'une couche réalisée en un matériau assurant une bonne transmission des ondes ultrasonores, cette couche présentant, à l'opposé dudit élément piézoélectrique, une surface de révolution dont la génératrice présente une forme incurvée et dont l'axe directeur correspond à l'axe de rotation du transducteur, cette surface étant destinée à venir au contact de la structure à examiner pendant la rotation du transducteur. Cette forme incurvée pourra être circulaire de manière à obtenir une forme torique ou une forme sphérique.

Grâce à ces dispositions, on supprime la présence de tout bord agressif dans la partie transducteur, pouvant venir au contact de l'oeil. On résout ainsi le premier problème des sondes ouvertes, car le transducteur ne sera plus un organe susceptible de venir endommager l'oeil et ce, même en cas de faux mouvement du clinicien. D'autre part, la matière utilisée est telle qu'elle supporte tous les protocoles de décontamination par trempages.

Le contact direct transducteur/tissu à examiner permet d'éviter les imprécisions précédemment évoquées. Seule l'application d'un gel sur les tissus est nécessaire pour assurer une bonne transmission des ultrasons.

Les fréquences pénétrant effectivement dans les tissus sont bien celles émises par le transducteur.

Des modes d'exécution de l'invention seront décrits ci-après, à titre d'exemples non limitatifs, avec référence aux dessins annexés dans lesquels :
La figure 1 est une coupe axiale d'une sonde à transducteur sphérique ;
La figure 2 est une coupe axiale à 90° de la coupe représentée figure 1 ;
La figure 3 est une coupe à échelle réduite d'une sonde du type de celle représentée figures 1 et 2 appliquée sur l'oeil d'un patient ;
La figure 4 est une représentation schématique d'une variante d'exécution de la sonde selon l'invention.

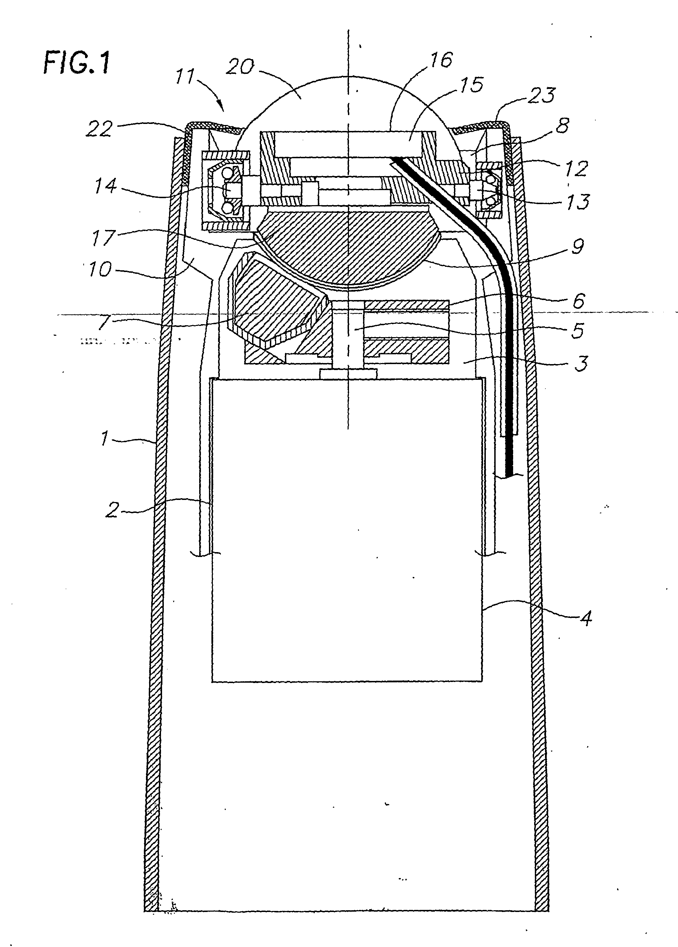

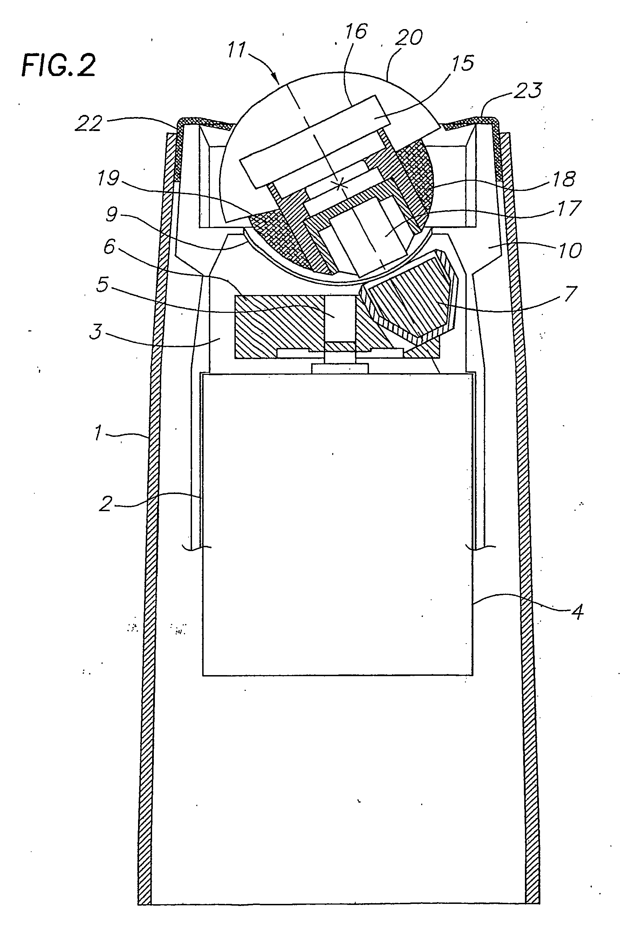

Dans l'exemple illustré sur les figures 1 et 2, la sonde présente un corps tubulaire à double paroi, partiellement représenté, comportant une paroi extérieure 1, par exemple en acier inoxydable, et une paroi intérieure 2, par exemple en matière plastique.

La paroi intérieure 2 délimite deux compartiments successifs, à savoir :
- un compartiment postérieur 3, dans lequel est logé un moto-réducteur coaxial 4, dont l'arbre central 5 entraîne en rotation un plateau tournant d'entraînement 6 portant un aimant permanent 7, et
- un compartiment antérieur 8, ouvert à son extrémité opposée au compartiment postérieur 3.

La séparation entre ces deux compartiments 3, 8 est assurée par une cloison sphérique 9, venue de moulage ou d'usinage avec la paroi intérieure 2 et dont la concavité est orientée vers le compartiment antérieur 8. Cette cloison sphérique 9 se trouve dans le prolongement d'un collet intérieur 10 formant un rétrécissement entre les deux chambres 3, 8.

L'aimant permanent 7, logé dans une cavité du plateau tournant 6, présente une forme cylindrotronconique axée obliquement par rapport à l'axe d'entraînement 5 du moteur 4 et dont la grande base s'étend tangentiellement à la cloison sphérique 9.

A l'intérieur de la chambre antérieure 8, est monté rotatif un transducteur sphérique 11 comportant une pièce principale de support 12 de forme sensiblement cylindrique à alésage étagé, montée rotative sur la paroi intérieure 2 grâce à deux tourillons coaxiaux 13, 14 axés perpendiculairement à l'axe principal de la pièce 12. Dans cet exemple, les deux tourillons 13, 14 sont portés par des roulements montés dans des logements cylindriques coaxiaux prévus dans la paroi intérieure 2.

Cette pièce principale de support 12 comprend, d'un côté à une distance prédéterminée de l'axe des deux tourillons 13, 14, un décrochement d'alésage délimitant une cavité ouverte vers l'extérieur dans laquelle est disposé un ensemble piézoélectrique 15 présentant un pouvoir de focalisation vers l'extérieur et dont sa forme extérieure 20 est sphérique.

Du côté de l'axe des tourillons opposé à l'élément 15, la pièce principale de support forme une chape qui délimite une cavité dans laquelle s'engage un aimant permanent d'entraînement 17 possédant une surface sphérique axée perpendiculairement à l'axe des tourillons 13, 14, épousant ainsi la forme de la cloison sphérique 9.

Sur les côtés latéraux de la pièce principale de support 12, qui s'étendent parallèlement à l'axe des tourillons 13, 14, sont fixés deux aimants latéraux respectifs 18, 19 (figure 2), destinés à coopérer avec un détecteur à effet Hall pour déterminer la position angulaire du transducteur 11. Ces aimants latéraux 18, 19 présentent une forme extérieure sphérique concentrique au transducteur 11.

Conformément à l'invention, la partie antérieure du transducteur 11 (située du côté de l'élément piézoélectrique, par rapport à l'axe des tourillons) est revêtue d'une couche en matière moulée 20 à haut pouvoir de transmission des ondes ultrasonores émises par l'ensemble piézoélectrique 15.

Dans cet exemple, cette couche 20, qui présente une surface extérieure sphérique coaxiale au transducteur 11, s'interrompt à une hauteur d'environ 5° par rapport au plan équatorial du transducteur 11. Cette couche 20 doit enrober suffisamment le transducteur pour assurer le contact avec les structures à explorer pendant toute la rotation.

L'étanchéité entre les parois extérieure 7 et intérieure 2, au voisinage de l'orifice, est assurée par un joint par exemple en élastomère 22, de forme cylindrique comportant une collerette radiale 23 orientée vers l'intérieur qui vient en recouvrement de l'extrémité de la paroi intérieure 2 pour venir en appui, par son bord intérieur, sur la surface sphérique de la couche 20.

Ce joint 22 permet donc d'éviter l'introduction de matière liquide ou solide à l'intérieur de la chambre antérieure 8 de la sonde 11.

Grâce aux dispositions précédemment décrites, en alimentant le moteur 4 en énergie électrique, on provoque la rotation du plateau tournant 6 et donc de l'aimant qui effectue une trajectoire circulaire autour de l'axe longitudinal de la sonde, au voisinage immédiat de la cloison sphérique 9.

Sous l'effet du champ magnétique tournant engendré par l'aimant 7, l'aimant permanent 17 se trouve soumis à une force d'attraction/répulsion qui provoque un mouvement de rotation alternatif du transducteur sphérique 11 autour de l'axe des tourillons 13, 14. Le transducteur 11 effectue un balayage sectoriel dont la position angulaire se trouve détectée grâce à l'action des aimants latéraux 18, 19 sur le détecteur à effet Hall.

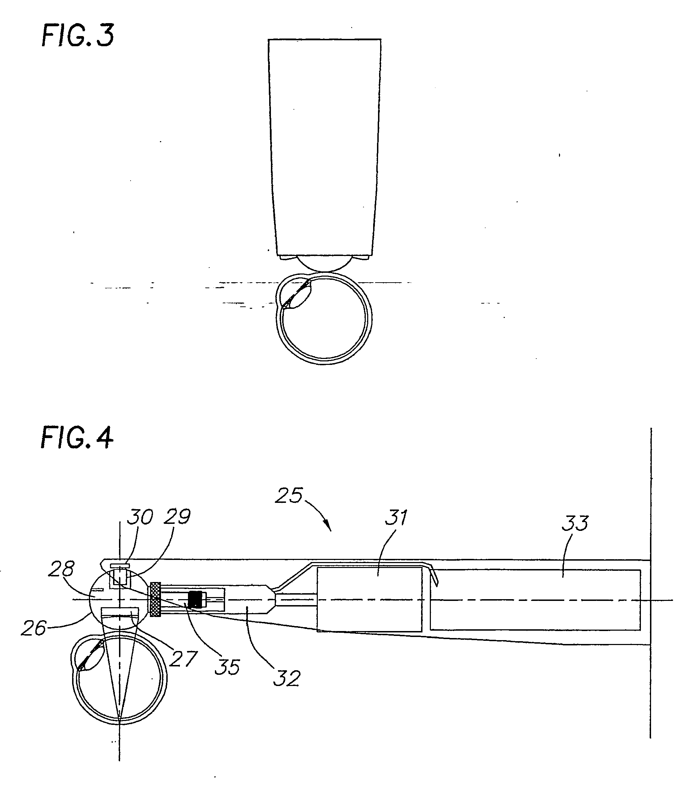

La partie antérieure du transducteur sphérique 11, qui ressort de la collerette 23 du joint en élastomère 22, peut être mise directement en contact avec l'oeil, de la façon indiquée sur la figure 3. Seule une légère couche de gel peut être appliquée sur l'oeil pour assurer une bonne transmission des ultrasons et améliorer le glissement entré l'oeil et le transducteur sphérique.

Bien entendu, l'invention ne se limite pas au mode d'exécution précédemment décrit.

Ainsi, par exemple, la sonde 25 (figure 4) pourra comprendre un transducteur sphérique 26 monté rotatif autour d'un axe parallèle à l'axe longitudinal du corps de la sonde 25.

Dans ce cas, l'élément piézoélectrique 27 pourra être axé perpendiculairement à l'axe de rotation du transducteur 26 comme illustré sur la figure 4.

Dans ce cas, l'ensemble piézoélectrique 27 est enrobé dans une pièce sphérique 28 en une matière assurant une bonne transmission des ondes ultrasonores.

L'entraînement en rotation de l'ensemble 27 est ici assuré au moyen d'un moteur 31 coaxial à la sonde et dont l'arbre de sortie, relié au transducteur grâce à un élément de transmission 32, assure la fixation mécanique de la pièce sphérique 28.

La connexion électrique de l'élément actif est réalisée par le connecteur à vis 35, type SMC ou équivalent, accessible à travers les parties évidées de l'élément de transmission 32. Un aimant permanent 29, faisant face à un capteur à effet Hall 30 solidaire du boîtier de la sonde 25, permet de renvoyer l'information de position à la carte 33 d'asservissement du moteur pour un mouvement alternatif d'aller/retour.

Compte tenu du fait que le secteur angulaire balayé par le transducteur 27 est axé perpendiculairement à l'axe de la sonde 25, l'extrémité antérieure du corps de la sonde 25 se termine d'un côté en biseau légèrement incurvé de manière à délimiter une ouverture oblique et à découvrir la zone utile du transducteur 27 qui devra être appliquée sur l'oeil. L'avantage de cette solution consiste en ce que, lors d'une échographie, la vision de l'oeil du patient par l'opérateur ne se trouve que très partiellement masquée par la sonde 25 (uniquement par son extrémité antérieure). La main tenant la sonde 25 sera en dehors du champ de vision, ce qui n'était pas le cas dans l'exemple précédemment décrit.

Par ailleurs, les mouvements du transducteur ne sont pas limités à de simples mouvements de rotation alternatifs. En effet, ces mouvements pourront être de type arciforme. Dans ce cas, le transducteur pourra être monté sur un dispositif d'actionnement par exemple du type de celui qui se trouve décrit dans la demande de brevet no FR2839543 du 7 mai 2002, au nom de la Demanderesse.

Dans ce cas, la forme sphérique du transducteur permet de limiter considérablement le risque d'accident.

## Revendications

1. Sonde échographique à balayage sectoriel comprenant un corps tubulaire (1, 2) logeant au moins partiellement dans son extrémité antérieure, un transducteur (11) conçu de manière à émettre une onde ultrasonore incidente focalisée en direction des structures à examiner et à recevoir des ondes ultrasonores engendrées par ces structures sous l'effet de cette onde incidente, ce transducteur (11) étant monté rotatif à l'intérieur de la sonde autour d'un axe de rotation et étant couplé à des moyens d'actionnement (4) de manière à pouvoir effectuer des déplacements au moins partiellement en rotation par rapport à la sonde en vue d'obtenir un balayage sectoriel de la structure à examiner,
le transducteur (11) comprenant un élément piézoélectrique (15) présentant un pouvoir de focalisation des faisceaux émis et, dont la partie antérieure est revêtue d'une couche (20) réalisée en un matériau assurant une bonne transmission des ondes ultrasonores, cette couche (20) présentant, à l'opposé dudit élément piézoélectrique, une surface extérieure de révolution convexe dont la génératrice présente une forme incurvée et dont l'axe directeur correspond à l'axe de rotation du transducteur de manière à pouvoir venir au contact de la structure à examiner pendant la rotation du transducteur (11).

2. Sonde selon la revendication 1,
**caractérisée en ce que** la susdite génératrice présente une forme circulaire.

3. Sonde selon l'une des revendications 1 et 2,
**caractérisée en ce que** la susdite surface de révolution est sphérique.

4. Sonde selon l'une des revendications précédentes,
**caractérisée en ce que** le transducteur (11) comprend un aimant permanent (18, 19) coopérant avec un capteur à effet Hall solidaire du corps pour assurer la détection de la position dudit transducteur (11).

5. Sonde selon l'une des revendications précédentes,
**caractérisée en ce que** le transducteur (11) est monté rotatif sur le corps tubulaire selon un axe de rotation et comprend un premier aimant permanent (17) situé à l'opposé de l'élément piézoélectrique (15) par rapport audit axe, l'actionnement dudit transducteur (11) étant assuré sans contact au moyen d'un second aimant permanent (7) monté sur un plateau d'entraînement rotatif (6) qui effectue une trajectoire circulaire axée perpendiculairement audit axe de rotation.

6. Sonde selon la revendication 5,
**caractérisée en ce que** le susdit transducteur (11) et le susdit plateau d'entraînement (6) muni du susdit second aimant permanent (7) sont respectivement disposés dans deux compartiments (3, 8) du corps séparés par une cloison (9).

7. Sonde selon la revendication 6,
**caractérisée en ce que** la susdite cloison (9) présente une forme sphérique concentrique au susdit transducteur (11).

8. Sonde selon l'une des revendications précédentes,
**caractérisée en ce que** le susdit corps tubulaire est à double parois, l'étanchéité entre les deux parois (1, 2) au niveau de l'orifice du corps traversé par le transducteur (11) est assurée par un joint cylindrique (22) comportant une collerette radiale (23) orientée vers l'intérieur dont le bord intérieur vient en appui sur la surface sphérique du transducteur (11).

9. Sonde selon l'une des revendications 1 à 3,
**caractérisée en ce qu'**elle comprend un transducteur (26) au moins partiellement sphérique monté autour d'un axe parallèle à l'axe longitudinal du corps (25) de la sonde, ce transducteur comportant un élément piézoélectrique (27) axé perpendiculairement à l'axe de rotation du transducteur (26) ; l'extrémité antérieure du corps de la sonde se terminant en biseau de manière à délimiter une ouverture oblique découvrant une zone utile du transducteur (26) axée transversalement à l'axe longitudinal du corps (25).

10. Sonde selon l'une des revendications 1 à 8,
**caractérisée en ce que** le susdit transducteur (11, 26) est monté sur un mécanisme d'actionnement permettant d'obtenir un balayage arciforme.

## Claims

1. An echographic probe with sector scanning comprising a tubular body (1, 2), housing at least partly in its front end a transducer (11) designed to emit an incident ultrasonic wave focused towards the structures to be examined and to receive ultrasonic waves generated by these structures under the effect of this incident wave, this transducer (11) being rotatably mounted inside the probe around an axis of rotation and being coupled with actuating means (4) so as to be able to perform displacements at least partly in rotation relatively to the probe in order to obtain a sector scan of the structure to be examined,
the transducer (11) comprising a piezoelectric element (15) having power for focusing the emitted beams and whose outer part is coated with a layer (20) made in a material providing good transmission of ultrasonic waves, this layer (20) having, opposite to said piezoelectric component, a convex axisymmetric outer surface, the generatrix of which has a curved shape and the director axis of which corresponds to the axis of rotation of the transducer so as to be able to come into contact with the structure to be examined during the rotation of the transducer (11).

2. The probe according to claim 1,
**characterized in that** the aforesaid generatrix has a circular shape.

3. The probe according to any of claims 1 and 2,
**characterized in that** the aforesaid axisymmetric surface is spherical.

4. The probe according to any of the preceding claims,
**characterized in that** the transducer (11) comprises a permanent magnet (18, 19) cooperating with a Hall effect sensor integral with the body in order to provide detection of the position of said transducer (11).

5. The probe according to any of the preceding claims,
**characterized in that** the transducer (11) is rotatably mounted on the tubular body along an axis of rotation and comprises a first permanent magnet (17) located opposite the piezoelectric component (15) relatively to said axis, contactless actuation of said transducer (11) being provided by means of a second permanent magnet (7) mounted on a rotary driving plate (6) which performs a circular trajectory centered perpendicularly to said axis of rotation.

6. The probe according to claim 5,
**characterized in that** the aforesaid transducer (11) and the aforesaid drive plate (6) provided with the aforesaid second permanent magnet (7) are positioned in two compartments (3, 8) of the body, separated by a partition (9), respectively.

7. The probe according to claim 6,
**characterized in that** the aforesaid partition (9) has a spherical shape concentric with the aforesaid transducer (11).

8. The probe according to any of the preceding claims,
**characterized in that** said tubular body is with double walls, the seal between both walls (1, 2) at the orifice of the body gone through by the transducer (11) is provided by a cylindrical gasket (22) including a radial flange (23) directed inwards, the interior edge of which will rest on the spherical surface of the transducer (11).

9. The probe according to any of claims 1 to 3,
**characterized in that** it comprises an at least partially spherical transducer (26) mounted around an axis parallel to the longitudinal axis of the body (25) of the probe, this transducer including a piezoelectric element (27) centered perpendicularly to the axis of rotation of the transducer (26); the front end of the body of the probe ending as a bevel in order to delimit an oblique aperture exposing a useful area of the transducer (26) centered transversally to the longitudinal axis of the body (25).

10. The probe according to any of claims 1 to 8,
**characterized in that** the aforesaid transducer (11, 26) is mounted on an actuation mechanism with which an arc-shaped scan may be obtained.

## Patentansprüche

1. Echografische Sonde zur sektoriellen Abtastung mit einem rohrförmigen Körper (1, 2), der in seinem vorderen Ende mindestens zum Teil einen Wandler (11) aufnimmt, welcher derart aufgebaut ist, dass er eine Ultraschallwelle aussendet, die in Richtung auf die zu untersuchenden Strukturen einfallend fokussiert ist, und Ultraschallwellen empfängt, welche von diesen Strukturen durch die Einwirkung dieser einfallenden Welle erzeugt werden, wobei dieser Wandler (11) um eine Rotationsachse drehbar im Inneren der Sonde angeordnet und an Antriebsmittel (4) derart angekoppelt ist, dass mindestens teilweise durch Rotation in Bezug auf die Sonde entstehende Lageveränderungen bewirkt werden in der Absicht, eine sektorielle Abtastung der zu untersuchenden Strukturen zu erhalten, wobei der Wandler (11) ein piezoelektrisches Element (15) umfasst, welches die Fähigkeit besitzt, ausgesendete Strahlenbündel zu fokussieren, und dessen Vorderteil mit einer Schicht (20) bedeckt ist, die aus einem eine gute Übertragung von Ultraschallwellen sicherstellenden Material besteht, wobei diese Schicht (20) an der von dem besagten piezoelektrischen Element abgewandten Seite eine konvexe äußere Rotationsfläche aufweist, deren Erzeugende eine gekrümmte Form besitzt und deren Bezugsachse der Rotationsachse des Wandlers entspricht derart, dass sie während der Rotation des Wandlers (11) mit der zu untersuchenden Struktur in Kontakt kommen kann.

2. Sonde nach Anspruch 1, **dadurch gekennzeichnet, dass** die obengenannte Erzeugende eine kreisförmige Gestalt aufweist.

3. Sonde nach einem der Ansprüche 1 und 2, **dadurch gekennzeichnet, dass** die obengenannte Rotationsfläche sphärisch ausgebildet ist.

4. Sonde nach einem der vorhergehenden Ansprüche, **dadurch gekennzeichnet, dass** der Wandler (11) einen Permanentmagneten (18, 19) umfasst, der mit einem Hall-Effekt-Sensor zusammenwirkt, welcher fest mit dem Körper verbunden ist, um die Erfassung der Stellung des besagten Wandlers (11) sicherzustellen.

5. Sonde nach einem der vorhergehenden Ansprüche, **dadurch gekennzeichnet, dass** der Wandler (11) um eine Rotationsachse drehbar am rohrförmigen Körper angeordnet ist und einen ersten Permanentmagneten (17) umfasst, der dem piezoelektrischen Element (15) in Bezug auf die besagte Achse gegenüberliegt, wobei der Antrieb des besagten Wandlers (11) kontaktlos durch einen zweiten Permanentmagneten (7) sichergestellt ist, welcher auf einer drehbaren Antriebsplatte (6) befestigt ist, die eine kreisförmige, senkrecht zur besagten Rotationsachse ausgerichtete Umlaufbahn bewirkt.

6. Sonde nach Anspruch 5, **dadurch gekennzeichnet, dass** der obengenannte Wandler (11) und die obengenannte, mit dem obengenannten zweiten Permanentmagneten (7) ausgerüstete Antriebsplatte (6) jeweils in zwei Teilräumen (3, 8) des Körpers angeordnet sind, welche durch eine Zwischenwand (9) voneinander getrennt sind.

7. Sonde nach Anspruch 6, **dadurch gekennzeichnet, dass** die besagte Zwischenwand (9) konzentrisch zu dem obengenannten Wandler (11) eine sphärische Form aufweist.

8. Sonde nach einem der vorhergehenden Ansprüche, **dadurch gekennzeichnet, dass** der obengenannte rohrförmige Körper doppelwandig ausgebildet ist, wobei die Dichtigkeit zwischen den beiden Wänden (1, 2) auf der Höhe der vom Wandler (11) durchsetzten Öffnung des Körpers durch eine zylindrische Dichtung (22) sichergestellt ist, welche einen nach innen gerichteten, radialen Kragen (23) besitzt, dessen innerer Rand sich an der sphärischen Oberfläche des Wandlers (11) abstützt.

9. Sonde nach einem der Ansprüche 1 bis 3, **dadurch gekennzeichnet, dass** sie einen mindestens teilweise sphärisch ausgebildeten Wandler (26) umfasst, der um eine zur Längsachse des Körpers (25) der Sonde parallele Achse angeordnet ist, wobei dieser Wandler ein piezoelektrisches Element (27) umfasst, das senkrecht zur Rotationsachse des Wandlers (26) ausgerichtet ist, wobei das vordere Ende des Körpers der Sonde in einer Abschrägung endet derart, dass eine schräge Öffnung begrenzt wird, welche einen quer zur Längsachse des Körpers (25) ausgerichteten, nutzbaren Bereich des Wandlers (26) freigibt.

10. Sonde nach einem der Ansprüche 1 bis 8, **dadurch gekennzeichnet, dass** der obengenannte Wandler (11, 26) auf einem Antriebsmechanismus angeordnet ist, welcher eine bogenförmige Abtastung ermöglicht.
